# EUROPÄISCHE PATENTANMELDUNG

(11) **EP 1 840 529 A2**
(43) Veröffentlichungstag der Anmeldung: **03.10.2007**
(21) Anmeldenummer: 06024128.8
(22) Anmeldetag: 21.11.2006
(51) Int. Cl.: G01F 1/66

(54) **Clamp-on Ultraschalldurchflußmeßgerät**

(30) Priorität: 30.03.2006 DE 102006015217
(71) Anmelder: Krohne AG, 4019 Basel (CH)
(72) Erfinder: Pors, Jan, 3262 EK Oudi-Bijerland (NL); v.d. Berg, Jeroen, 3342 AE Hendrik Ido Ambacht (NL); Molenaar, Marcel, 3311 ZG Dordrecht (NL); Hogendoorn, Jankees, 4211 BG Spijk (NL)
(74) Vertreter: Gesthuysen, von Rohr & Eggert

(57) **Zusammenfassung**

Die Erfindung betrifft ein Ultraschalldurchflußmeßgerät zur Messung des Durchflusses durch eine von einem Medium durchströmte Leitung (1), mit wenigstens einem Meßwandler (2) und einem Führungsrahmen (3) zum Führen und Halten des Meßwandlers (2), Erfindungsgemäß ist eine elektrische Verbindungsbox (12) vorgesehen, die einen Anschluß (13) für ein Kabel (14) zu dem Meßwandler (2) und einen Anschluß (15) für ein Kabel (16) zur Meßgeräteelektronik aufweist. Damit wird insbesondere bei Clamp-on-Ultraschalldurchflußmeßgeräten eine einfache Verkabelung erzielt.

## Beschreibung

Die Erfindung betrifft ein Ultraschalldurchflußmeßgerät, insbesondere ein Clamp-on-Ultraschalldurchflußmeßgerät, zur Messung des Durchflusses durch eine von einem Medium durchströmte Leitung, mit wenigstens einem Meßwandler und einem Führungsrahmen zum Führen und Halten des Meßwandlers.

Clamp-on-Ultraschalldurchflußmeßgeräte zeichnen sich dadurch aus, daß sie besonders einfach eingesetzt werden können: Im Gegensatz zu anderen Ultraschalldurchflußmeßgeräten, die in bestehende Rohrleitungssysteme fest integriert werden müssen, indem sie ein Stück der Rohrleitung ersetzen, können Clamp-on-Durchflußmeßgeräte einfach von außen auf einen geeigneten Leitungsabschnitt des Rohrleitungssystems aufgesetzt werden. Dieser Leitungsabschnitt, an dem das Clamp-on-Ultraschalldurchflußmeßgerät angebracht ist, wird damit quasi zur Meßleitung, ohne daß es einer eigenen separaten Meßleitung bedarf, die in das Rohrleitungssystem eingesetzt werden muß. Dies macht die Verwendung von Clamp-on-Ultraschalldurchflußmeßgeräten einfach und kostengünstig.

Problematisch bei Clamp-on-Ultraschalldurchflußmeßgeräten ist jedoch die korrekte Anbringung des Meßgeräts an einer Leitung, insbesondere hinsichtlich der korrekten Anordnung und Ausrichtung der Meßwandler, die bei einem Ultraschalldurchflußmeßgerät in der Regel durch zwei in Längsrichtung der Leitung im Abstand voneinander angeordnete Meßwandler gebildet werden. Neben einer entsprechende Problematik bei der ersten Anbringung des Clamp-on-Ultraschalldurchflußmeßgeräts auf der Leitung ergibt sich das Problem, daß selbst bei korrekt angeordnetem und ausgerichteten Meßgerät dieser Zustand wieder verloren werden kann, wenn die Meßwandler, z. B. zu Wartungszwecken, von der Leitung entfernt werden müssen.

Insbesondere bei Clamp-on-Ultraschalldurchflußmeßgeräten ist problematisch, daß jeder Meßwandler mit einem eigenen Kabel versehen ist, das ihn mit der Meßgeräteelektronik verbindet. Dies ist insbesondere bei der Installation eines Clamp-on-Ultraschalldurchflußmeßgeräts hinderlich, aber auch bei dem Abnehmen und Wiederanbringen des Clamp-on-Ultraschalldurchflußmeßgeräts im Rahmen einer Wartung oder Reparatur.

Dementsprechend ist es die Aufgabe der Erfindung, ein derartiges Ultraschalldurchflußmeßgerät, insbesondere Clamp-on-Ultraschalldurchflußmeßgerät, anzugeben, das hinsichtlich seiner Verkabelung einfach zu handhaben ist.

Ausgehend von dem eingangs beschriebenen Ultraschalldurchflußmeßgerät ist diese Aufgabe dadurch gelöst, daß eine elektrische Verbindungsbox vorgesehen ist, die einen Anschluß für ein Kabel zu dem Meßwandler und einen Anschluß für ein Kabel zur Meßgeräteelelctronik aufweist.

Das erfindungsgemäße Vorsehen einer elektrischen Verbindungsbox ist insofern vorteilhaft, als daß die Verbindungsbox zusammen mit dem Führungsrahmen und dem Meßwandler gehandhabt werden kann, z. B. von der Leitung entfernt werden kann, ohne das Kabel vom Meßwandler zur Verbindungsbox zu entfernen, während jedoch kein Kabel von der Meßgerätelektronik zur Verbindungsbox angeschlossen ist.

Grundsätzlich können die Anschlüsse für ein Kabel zu dem Meßwandler bzw. für ein Kabel zur Meßgeräteelektronik auf verschiedene Arten ausgebildet sein. Gemäß einer bevorzugten Weiterbildung der Erfindung ist jedoch vorgesehen, daß für den Anschluß für ein Kabel zu dem Meßwandler und/oder für den Anschluß für ein Kabel zur Meßgeräteelektronik jeweils eine Steckverbindung vorgesehen ist.

Besonders vorteilhaft ist es, wenn das Kabel zu dem Meßwandler innerhalb des Führungsrahmens verläuft. Auf diese Weise besteht praktisch kein Risiko, daß dieses Kabel bei der Handhabung des Ultraschalldurchflußmeßgeräts, z. B. beim Abnehmen desselben von der Leitung, beeinträchtigt wird.

Eine besonders einfache Handhabung des Ultraschalldurchflußmeßgeräts ergibt sich insbesondere auch dann, wenn gemäß einer bevorzugten Weiterbildung der Erfindung vorgesehen ist, daß die elektrische Verbindungsbox an dem Führungsrahmen befestigt ist.

Schließlich ist gemäß einer bevorzugten Weiterbildung der Erfindung vorgesehen, daß zwei Meßwandler vorgesehen sind, wobei von der Verbindungsbox jeweils ein separates Kabel zu jedem der Meßwandler läuft, während zur Meßgeräteelektronik nur ein einziges Kabel vorhanden ist. Zum Entfernen des Ultraschalldurchflußmeßgeräts von der Leitung muß damit nur ein einziger Anschluß gelöst werden, nämlich der Anschluß des von der Meßgeräteelektronik herkommenden Kabels an der Verbindungsbox, was die Handhabung besonders einfach macht, insbesondere nämlich vermeidet, mehr als ein einziges Kabel handhaben zu müssen.

Gemäß einer bevorzugten Weiterbildung der Erfindung ist eine Fixiereinrichtung vorgesehen, mittels derer der Meßwandler auf die Leitung zu bzw. von der Leitung wegbewegt und an dem Führungsrahmen fixiert bzw. von diesem gelöst werden kann.

Damit ist also eine zwei Funktionen übernehmende Fixiereinrichtung vorgesehen, mittels derer der Meßwandler, vorzugsweise senkrecht, auf die Leitung zubewegt und schließlich gegen diese gedrückt werden kann, um einen guten akustischen Kontakt zwischen Meßwandler und Leitung zu gewährleisten. Mit der Fixiereinrichtung kann der Meßwandler natürlich auch wieder von der Leitung wegbewegt werden, so daß er die Leitung nicht mehr berührt. Als zweite Funktion wird mittels der Fixiereinrichtung erreicht, daß der Meßwandler an einem Führungsrahmen fixiert bzw. auch wieder von diesem gelöst werden kann.

Gemäß einer bevorzugten Weiterbildung der Erfindung ist dabei vorgesehen, daß das Ultraschalldurchflußmeßgerät mittels des Führungsrahmens an der Leitung befestigbar ist. Dabei kann einerseits vorgesehen sein, daß die Befestigung unmittelbar über den Führungsrahmen erfolgt, es kann jedoch auch vorgesehen sein, daß der Führungsrahmen, z. B. beweglich, an Befestigungseinrichtungen befestigt ist, die ihrerseits an der Leitung befestigt sind.

Gemäß einer bevorzugten Weiterbildung der Erfindung ist ferner vorgesehen, daß die Fixiereinrichtung derart ausgestaltet und angeordnet ist, daß der Meßwandler bei einer Bewegung auf die Leitung zu gleichzeitig an dem Führungsrahmen fixiert und bei einer Bewegung von der Leitung weg gleichzeitig von dem Führungsrahmen gelöst wird.

Das Lösen des Meßwandlers vom Führungsrahmen ist bei der vorliegenden Erfindung so zu verstehen, daß der Meßwandler weiterhin, vorzugsweise unverlierbar, an dem Führungsrahmen befestigt bleibt, jedoch auf diesem bewegbar ist. Insbesondere ist gemäß einer bevorzugten Weiterbildung der Erfindung vorgesehen, daß der Meßwandler in dem von dem Führungsrahmen gelösten Zustand auf dem Führungsrahmen, vorzugsweise in dessen Längsrichtung, verschiebbar ist und dabei von dem Führungsrahmen geführt wird.

Auf diese Weise kann der Meßwandler innerhalb eines an einer Leitung befestigten Führungsrahmens z. B. in Längsrichtung der Leitung verschoben werden, um den richtigen Ort der Positionierung des Meßwandlers zu finden. Dort kann der Meßwandler dann an dem Führungsrahmen fixiert werden, wobei gleichzeitig eine Bewegung auf die Leitung zu erfolgt, so daß der Meßwandler schließlich mit der Leitung in Kontakt kommt, um einen guten akustischen Übergang zwischen dem Meßwandler und der Leitung und somit auch zu dem durch die Leitung strömenden Medium zu erzielen.

Grundsätzlich kann die Fixierrichtung in unterschiedlicher Weise ausgebildet sein, um die zuvor beschriebenen Eigenschaften aufzuweisen, Gemäß einer bevorzugten Weiterbildung der Erfindung ist jedoch vorgesehen, daß die Fixiereinrichtung einen Drehknopf aufweist, durch dessen Drehung in die eine Richtung der Meßwandler auf die Leitung zubewegt und gleichzeitig an dem Führungsrahmen fixiert wird und durch dessen Drehung in die andere Richtung der Meßwandler von der Leitung wegbewegt und gleichzeitig von dem Führungsrahmen gelöst wird.

Gemäß einer bevorzugten Weiterbildung der Erfindung kann auch vorgesehen sein, daß die Fixiereinrichtung den Meßwandler an dem Führungsrahmen fixiert, indem sie den Meßwandler oder eine an dem Meßwandler vorgesehene Klemmeinrichtung mit dem Führungsrahmen verklemmt. Vorzugsweise ist in diesem Zusammenhang vorgesehen, daß die Fixiereinrichtung innerhalb einer in dem Führungsrahmen vorgesehenen Führungsnut verläuft und zum Fixieren des Meßwandlers eine Verklemmung auf den die Nut seitlich begrenzenden Wänden des Führungsrahmens erfolgt.

In diesem Zusammenhang ist gemäß einer bevorzugten Weiterbildung der Erfindung grundsätzlich vorgesehen, daß an dem Meßwandler eine Führungseinrichtung angeordnet ist, die mit dem Führungsrahmen derart zusammenwirkt, daß die Längsverschiebbarkeit des Meßwandlers in seinem gelösten Zustand gewährleistet ist. Dabei kann z. B. auch in dem Meßwandler oder in einer an dem Meßwandler vorgesehenen Einrichtung eine Nut vorgesehen sein, die in einem korrespondierenden Gegenstück an dem Führungsrahmen läuft.

Gemäß einer bevorzugten Weiterbildung der Erfindung ist weiterhin vorgesehen, daß der Führungsrahmen mit an ihm fixiertem Meßwandler von der Leitung lösbar und danach wieder an dieser befestigbar ist. Das bedeutet, daß der Meßwandler auch im von der Leitung entfernten Zustand des Führungsrahmens in vorbestimmter Weise im Führungsrahmen fixiert bleibt. Dies ist insbesondere dann von Bedeutung, wenn gemäß einer bevorzugten Weiterbildung der Erfindung zwei Meßwandler vorgesehen sind, die jeweils mittels einer eigenen Fixiereinrichtung in vorbestimmtem Abstand voneinander an dem Führungsrahmen fixiert sind, wobei der Führungsrahmen in diesem Zustand von der Leitung lösbar und danach wieder an dieser befestigbar ist. Damit ist insbesondere der Vorteil verbunden, daß nach einer erstmaligen korrekten Ausrichtung der Meßwandler zueinander diese Anordnung auch nach einer Reparatur oder einer Wartung, zu dem die Meßwandler von der Leitung entfernt werden müssen, erhalten bleibt.

Nachfolgend wird die Erfindung unter Bezugnahme auf die Zeichnung anhand bevorzugter Ausführungsbeispiele im einzelnen erläutert. In der Zeichnung zeigt
- Fig. 1a, b: ein Ultraschalldurchflußmeßgerät gemäß einem ersten bevorzugten Ausführungsbeispiel im Schnitt,
- Fig. 2a, b: das Ultraschalldurchflußmeßgerät gemäß dem ersten bevorzugten Ausführungsbeispiel der Erfindung in einer perspektivischen Ansicht, teilweise ebenfalls im Schnitt,
- Fig. 3: ein Ultraschalldurchflußmeßgerät gemäß einem zweiten bevorzugten Ausführungsbeispiel der Erfindung mit einer elektrischen Verbindungsbox vor deren Anbringung an dem Führungsrahmen,
- Fig. 4: das Ultraschalldurchflußmeßgerät gemäß dem zweiten bevorzugten Ausführungsbeispiel der Erfindung mit an den Führungsrahmen angebrachter elektrischer Verbindungsbox und
- Fig. 5: das Ultraschalldurchflußmeßgerät gemäß dem zweiten bevorzugten Ausführungsbeispiel der Erfindung in vollständig zusammengebautem Zustand.

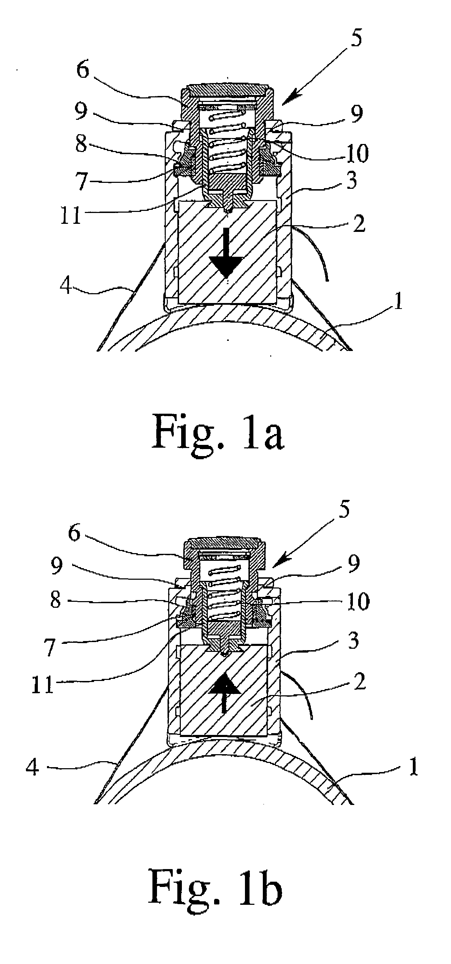

Aus den Fig. 1a und 1b ist ein Clamp-on-Ultraschalldurchflußmeßgerät gemäß einem ersten bevorzugten Ausführungsbeispiel der Erfindung ersichtlich, das auf einer von einem Medium durchströmbaren Leitung 1 befestigt ist. Das Clamp-on-Ultraschalldurchflußmeßgerät gemäß dem ersten bevorzugten Ausführungsbeispiel der Erfindung weist einen Meßwandler 2 und einen Führungsrahmen 3 auf, der zum Führen und Halten des Meßwandlers 2 dient und mittels eines Befestigungsbandes 4 an der Leitung 1 befestigt ist.

Bei dem Clamp-on-Ultraschalldurchflußmeßgerät gemäß dem vorliegend beschriebenen ersten bevorzugten Ausführungsbeispiel der Erfindung ist nun eine Fixiereinrichtung 5 vorgesehen, mittels derer der Meßwandler 2 senkrecht zur Längsachse der Leitung 1 auf diese zubewegt (Fig. 1a) und ebenfalls senkrecht zur Längsachse der Leitung 1 von dieser wegbewegt werden kann (Fig. lb). Gleichzeitig bewirkt die Fixiereinrichtung 5, daß bei der Bewegung auf die Leitung 1 zu eine Fixierung des Meßwandlers 2 an dem Führungsrahmen 3 erfolgt, während bei der Bewegung des Meßwandlers 2 von der Leitung 1 weg der Meßwandler 2 von dem Führungsrahmen 3 gelöst wird. In diesem gelösten Zustand ist der Meßwandler 2 dann parallel zur Längsachse der Leitung 1 verschiebbar, also senkrecht in die Zeichnungsebene hinein bzw. aus dieser heraus.

Die Fixiereinrichtung weist einen Drehknopf 6 auf, der über ein Gewinde 7 mit einer in dem Führungsrahmen 3 parallel zur Längsachse der Leitung 1 geführten Klemmeinrichtung 8 zusammenwirkt. Der Meßwandler 2 ist mit seiner Oberseite an dem Drehknopf 6 befestigt, wobei ein Drehen des Drehknopfes 6 im Uhrzeigersinn bzw. entgegengesetzt dazu, dazu führt, daß der Meßwandler 2 auf die Leitung 1 gedrückt wird bzw. von dieser abgehoben wird. Die Oberseite des Drehknopfes 6 weist einen größeren Durchmesser auf als der Mittelteil des Drehknopfes 6, so daß der Drehknopf 6 mit seinem Oberteil auf den Seitenwänden 9 des Führungsrahmens 3 aufliegt, die eine parallel zur Längsachse der Leitung 1 verlaufende Nut in dem Führungsrahmen 2 begrenzen.

Wenn der Drehknopf 6 im Uhrzeigersinn in die Klemmeinrichtung 8 eingeschraubt wird, so drückt diese von unten gegen die Seitenwände 9, so daß die Fixiereinrichtung 5 über den oberen Bereich des Drehknopfes 6 einerseits und die auf der anderen Seite der Seitenwände 9 vorgesehene Klemmeinrichtung 8 andererseits an dem Führungsrahmen 2 verklemmt wird. Darüber hinaus erfolgt durch das Einschrauben des Drehknopfes 6 in die Klemmeinrichtung 8 eine Bewegung des Meßwandlers 2 auf die Leitung 1 zu, so daß der Meßwandler 2 im Ergebnis fest auf die Leitung 1 drückt, was einen guten akustischen Übergang gewährleistet. In diesem Zustand läßt sich der Meßwandler 2 nicht mehr bewegen, insbesondere auch nicht in Längsrichtung des Führungsrahmens 3.

Um einen hinreichend hohen Anpreßdruck für die Befestigung des Meßwandlers 2 auf der Leitung 1 zu gewährleisten, ohne zu riskieren, daß der Meßwandler 2 durch das Andrücken auf die Leitung 1 beschädigt wird, ist innerhalb des Drehknopfes 6 eine Feder 10 vorgesehen. Diese Feder 10 drückt oben gegen die Oberseite des Drehknopfes 6 und wirkt unten mit einem in dem Drehknopf 6 senkrecht zur Längsachse des Führungsrahmens 3 beweglichen Federgehäuse 11 zusammen, über das der Meßwandler 2 an der Fixiereinrichtung 5 befestigt ist.

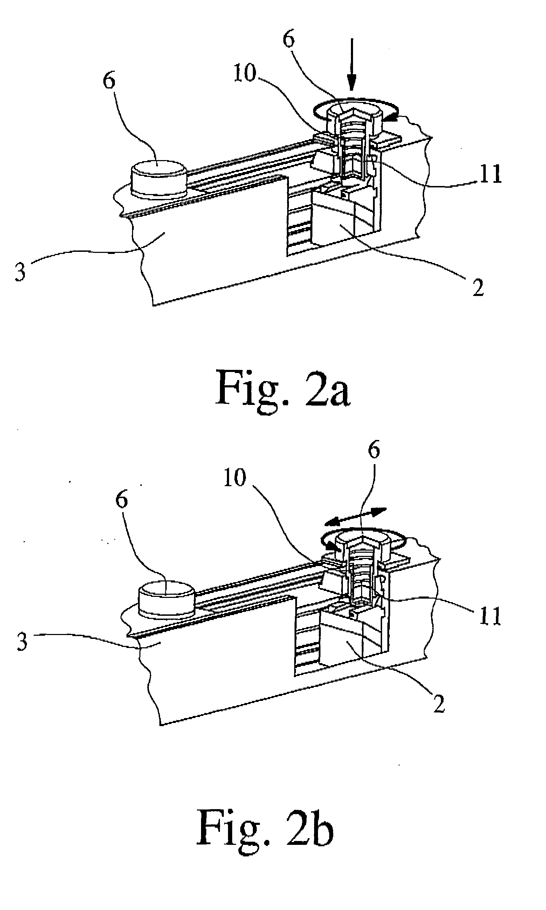

Wie aus den Fig. 2a und 2b nochmals ersichtlich, führt ein Drehen des Drehknopfes 6 im Uhrzeigersinn zu einer Fixierung des Meßwandlers 2 im Führungsrahmen 3 derart, daß keine Bewegung des Meßwandlers 2 in Längsrichtung mehr möglich ist, während der Meßwandler 2 fest auf die Leitung 1 gedrückt wird. Bei der Drehung des Drehknopfes im Gegenuhrzeigersinn wird der Meßwandler 2 von der Leitung 1 abgehoben und es ist eine Bewegung des Meßwandlers 2 in Längsrichtung des Führungsrahmens 3 möglich, z. B. um den Meßwandler 2 neu zu positionieren.

Wie ebenfalls aus den Fig. 2a und 2b ersichtlich, weist das Clamp-on-Ultraschalldurchflußmeßgerät gemäß dem vorliegend beschriebenen ersten bevorzugten Ausführungsbeispiel der Erfindung zwei Meßwandler 2 auf, wobei von dem linken Meßwandler nur der obere Teil des Drehknopfes 6 zu sehen ist. Bei zwei Meßwandlern 2 ergibt sich mit dem Clamp-on-Ultraschalldurchflußmeßgerät gemäß dem vorliegend beschriebenen ersten bevorzugten Ausführungsbeispiel der Erfindung der Vorteil, daß nach korrekter Positionierung der Meßwandler 2 diese an dem Führungsrahmen 3 fixiert werden können, wobei der Führungsrahmen 3 dann von der Leitung 1 abgenommen werden kann, ohne die korrekte Ausrichtung der Meßwandler 2 zueinander zu gefährden.

Im Ergebnis wird damit ein derartiges Clamp-on-Ultraschalldurchflußmeßgerät bereitgestellt, das auf einfache Weise, z. B. zu Wartungs- oder Reparaturzwecken, von der Leitung 1 gelöst werden kann, ohne beim Wiederanbringen an der Leitung 1 die Meßwandler 2 wieder aufwendig zueinander justieren zu müssen.

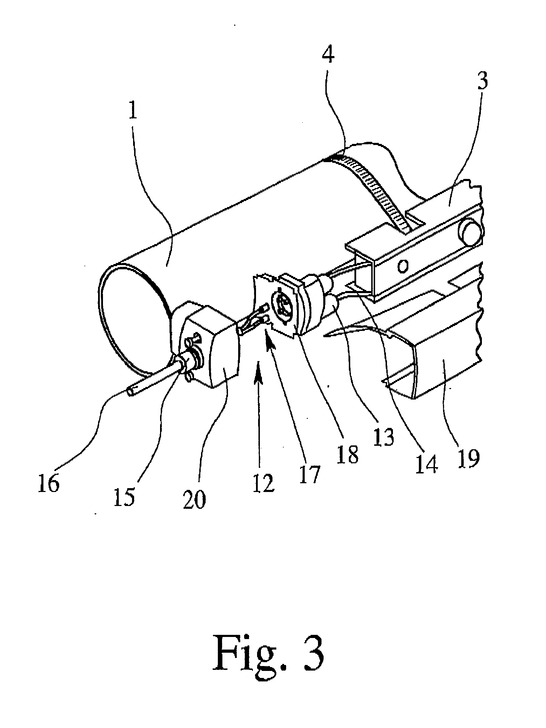

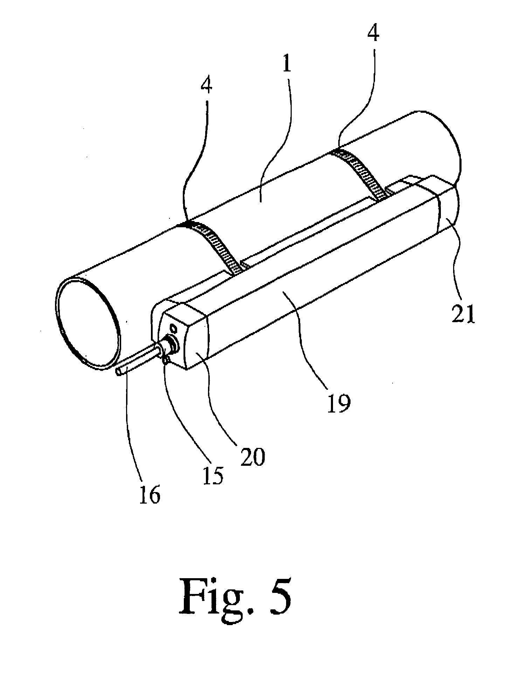

Bei einem Ultraschalldurchflußmeßgerät gemäß einem zweiten bevorzugten Ausführungsbeispiel der Erfindung, das aus den Fig. 3 bis 5 ersichtlich ist, ist nun im wesentlichen derselbe Aufbau vorgesehen, wie zuvor beschrieben. Zusätzlich ist hier jedoch eine elektrische Verbindungsbox 12 vorgesehen. Diese elektrische Verbindungsbox 12 weist zwei Anschlüsse 13 auf, die über jeweils ein Kabel 14 zu den Meßwandlern 2 führen. Außerdem weist die elektrische Verbindungsbox einen Anschluß 15 auf, der über ein Kabel 16 zu der nicht weiter dargestellten Meßgeräteelektronik führt. Die elektrische Verbindungsbox 12 ist derart aufgebaut, daß sie eine Steckverbindung 17 aufweist, die auf einfache Weise gelöst werden kann, um dann das gesamte Ultraschalldurchflußmeßgerät gemäß dem vorliegend beschriebenen zweiten bevorzugten Ausführungsbeispiel der Erfindung auf einfache Weise von der Leitung 1 lösen zu können.

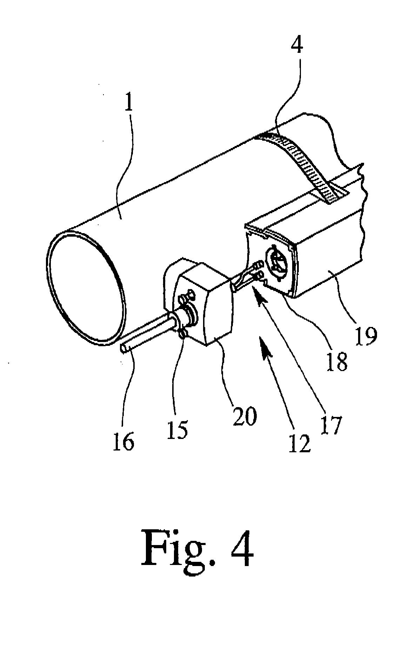

Wie insbesondere aus den Fig. 3 und 4 ersichtlich, ist die elektrische Verbindungsbox 12 zweiteilig aufgebaut, wobei das erste Teil 18 direkt an dem Führungsrahmen 3 befestigt wird. Danach erfolgt beim Zusammenbau des Ultraschalldurchflußmeßgeräts gemäß dem zweiten bevorzugten Ausführungsbeispiel der Erfindung das Aufsetzen eines Gerätedeckels 19, bevor das zweite Teil 20 der elektrischen Verbindungsbox 12 seitlich an das Ultraschalldurchflußmeßgerät angesetzt und mit dem ersten Teil 18 verschraubt wird, so daß der Gerätedeckel 19 seitlich verschlossen wird. Auf der gegenüberliegenden Seite wird der Gerätedeckel 19 durch eine entsprechende Kappe 21 ohne Kabeldurchführung verschlossen.

Damit wird ein Ultraschalldurchflußmeßgerät erzielt, das auch hinsichtlich seiner Verkabelung einfach zu handhaben ist, bei dem nämlich nur geringe Gefahr besteht, daß beim Installieren oder Abnehmen des Ultraschalldurchflußmeßgeräts von der Leitung 1 eines der Kabel 13, 14, 16 verletzt wird.

## Patentansprüche

1. Ultraschalldurchflußmeßgerät, insbesondere Clamp-on-Ultraschalldurchflußmeßgerät, zur Messung des Durchflusses durch eine von einem Medium durchströmte Leitung (1), mit wenigstens einem Meßwandler (2) und einem Führungsrahmen (3) zum Führen und Halten des Meßwandlers (2), **dadurch gekennzeichnet, daß** eine elektrische Verbindungsbox (12) vorgesehen ist, die einen Anschluß (13) für ein Kabel (14) zu dem Meßwandler (2) und einen Anschluß (15) für ein Kabel (16) zur Meßgeräteelektronik aufweist.

2. Ultraschalldurchflußmeßgerät nach Anspruch 1, **dadurch gekennzeichnet, daß** für den Anschluß (13) für das Kabel (14) zu dem Meßwandler (2) und/oder für den Anschluß (15) für das Kabel (16) zu der Meßgeräteelektronik jeweils eine Steckverbindung (17) vorgesehen ist.

3. Ultraschalldurchflußmeßgerät nach Anspruch 1 oder 2, **dadurch gekennzeichnet, daß** das Kabel (14) zu dem Meßwandler (2) innerhalb des Führungsrahmens (3) verläuft.

4. Ultraschalldurchflußmeßgerät nach einem der Ansprüche 1 bis 3, **dadurch gekennzeichnet, daß** die elektrische Verbindungsbox (12) an dem Führungsrahmen (3) befestigt ist.

5. Ultraschalldurchflußmeßgerät nach einem der Ansprüche 1 bis 4, **dadurch gekennzeichnet, daß** zwei Meßwandler (2) vorgesehen sind.
